# EUROPEAN PATENT SPECIFICATION

(11) **EP 1 406 540 B1**
(45) Date of publication and mention of the grant of the patent: **15.06.2011**
(21) Application number: 02784823.3
(22) Date of filing: 08.07.2002
(51) Int. Cl.: A61B 5/00, H04L 12/28

(54) **METHOD AND SYSTEM FOR CONTROLLING DATA INFORMATION BETWEEN TWO PORTABLE MEDICAL APPARATUSES**
VERFAHREN UND SYSTEM ZUR STEUERUNG VON DRAHTLOSEN DATENINFORMATIONEN ZWISCHEN ZWEI TRAGBAREN MEDIZINISCHEN VORRICHTUNGEN
PROCEDE ET SYSTEME DE CONTROLE D'INFORMATIONS DE DONNEES ENTRE DEUX APPAREILS MEDICAUX PORTATIFS

(30) Priority: 09.07.2001 DK 200101073
(43) Date of publication of application: 14.04.2004
(73) Proprietor: NOVO NORDISK A/S, 2880 Bagsværd (DK)
(72) Inventor: GRASKOV, Henning, DK-2880 Bagsværd (DK); HANSEN, Henrik, Egesborg, DK-2900 Hellerup (DK); EILERSEN, Michael, DK-2650 Hvidore (DK); LAV, Steffen, DK-2700 Brønshøj (DK)
(74) Representative: Kellberg, Lars
(86) International application number: PCT/DK2002/000474
(87) International publication number: WO 2003/005891

(56) References cited:
- EP-A- 0 519 137
- EP-A- 0 770 349
- WO-A-01/24690
- US-A- 5 528 323
- US-A- 6 093 146

## Description

The present invention relates to a method of controlling data information between two portable apparatuses, the use of the apparatuses including a first operation and a second operation, said portable apparatuses comprising a first apparatus for performing the first operation and a second apparatus for performing the second operation, where each apparatus has means for one or more of the following: storing, transmitting, receiving, processing and displaying data information, and where the two apparatuses have a number of interrelated positions during normal use.

The present invention also relates to a system for controlling data information between two portable apparatuses, the use of the apparatuses including a first operation and a second operation, said portable apparatuses comprising a first apparatus for performing the first operation and a second apparatus for performing the second operation, where each apparatus has means for one or more of the following: storing, transmitting, receiving, processing and displaying data information, and where the two apparatuses have a number of interrelated positions during normal use.

This invention is applicable to many uses including apparatuses used in connection with medical self-treatment(s), astma, hypertension, etc., but as a preferred example in the following the use relates to self-management of diabetes. Other examples are the administration and control of other hormone therapies. Yet another example is related to the administration of glycogen-like peptide 1 (GLP1) to type 2 diabetics. Other apparatuses (also non-medical) may be used in connection with the present invention just as well.

For a number of years it has been possible to purchase various devices for the treatment of diabetes, e.g. for injecting insulin, for measuring blood sugar (such a device is referred to as a BGM in the following), for withdrawing blood samples, and other accessories, the purpose of which is to enable the user to nurse his disease discretely and with a high standard of safety.

EP 0519137 discloses a system able to relaying physiological data from a patient to a receiving station. The data can be sent via infra red communication means from one apparatus to a second apparatus, and further via telephone lines from the second apparatus to further receiving stations. WO 0124690 describes a system for delivery of medicament an also for communication from the delivery apparatus to a network computer, according to the respective preambles of claims 1 and 19.

Many diabetic patients are elderly people who can easily get insecure with respect to the medical equipment. It is very reassuring and therefore also very important that the user can have feedback from the system and every operation is performed as smoothly as possible, which confirms to the user that everything is OK right from the technical function of the system to the user's physiological condition. This adds confidence to the user which, in the example of diabetes related equipment, contributes to improving the quality of life of users.

Also many young people need to assure themselves that the equipment is in order, i.e. calibrated, powered, updated and otherwise ready to be operated.

According to the invention the individual devices may be arranged for various respective functions relevant to the self-treatment of e.g. diabetes, such as: a lancet device, a body fluid analyser, one or more drug administration apparatuses for administering a predetermined dose of medication to the user. Further, there is a number of other aids which the diabetic patient uses, e.g. test strips for the blood analyser, needles, napkins for wiping off blood, extra insulin cartridge, glucose tablets, waste containers, etc.

An object of the present invention is to provide a method which assists a user in a transparent and very simple fashion by collecting data information relevant to the use of apparatuses so the user does not have to worry about these things and e.g. keep a separate log-book of any events, actions, etc.

An additional object is to obtain simple, efficient and relatively inexpensive exchange of relevant information between a number of relevant apparatuses where the user does not have to do anything but follow the normal use of the apparatuses. In the example of the user being a diabetic the user does not have to do anything but follow the normal steps of the self-treatment.

Another object is to provide automatic transmission of data information between a number of apparatuses that requires relatively small amount of energy.

These objects among others are achieved by a method of the aforementioned kind that further comprises the steps of:
- automatically storing at least first data information relevant to said first operation in said first apparatus,
- automatically storing at least second data information relevant to said second operation in said second apparatus, and
- automatically transmitting, via short-range communications means, data information relevant to at least one of said first and second operations between said first and second apparatuses when said apparatuses are mutually positioned in one of said number of interrelated positions.

Hereby, simplicity for the user is obtained, since the apparatuses relevant for the self-treatment automatically store and exchange data information as part of the normal use. Additionally, relevant data information is automatically exchanged between the first and second apparatuses, thereby enabling a single record of all relevant information to be stored e.g. in a single apparatus. This happens transparently and automatically without any user involvement other than a normal use of the apparatuses.

Additionally, by using short-range communications means arranged to communicate in an interrelated position relevant for the normal use, a very simple, efficient, and relatively inexpensive way of communicating may be obtained, since short-range communications means may be of a simplified design. The use of short-range communications means also reduces the amount of energy needed for communication, which is especially advantageous for portable apparatuses which normally have a limited power supply/source.

In this way, the user does not have to worry about collecting data information in a separate log-book, and, additionally, the data information may be collected in a single apparatus for further processing and/or use. In this way, a complete log-book is obtained in e.g. a single apparatus, which may be used by the user to obtain detailed information of trends, current and/or previous state(s), re-occurring events, e.g. that adverse effects relating to the self-treatment occurs every Sunday by using/analysing for behavioural and/or measured physiological patterns.

This also enhances the data quality of the data information and minimizes/eliminates the possibility of human error.

According to a preferred embodiment, the step of automatically transmitting data information between said first and second apparatuses comprises one or more of the following:
- checking a unique and individual apparatus identification number for each apparatus, and
- pairing a first apparatus and a second apparatus by linking their individual and unique identification number.

In this way a given first apparatus and at least a given second apparatus may be paired to form a single corresponding set. A given apparatus may check whether a communicating apparatus is a paired one and only initiate communication in the affirmative. Hereby, a given first apparatus may only exchange data information with at least a second apparatus e.g. belonging to the same user, the patient's physician or any other appropriate and approved apparatuses.

According to a preferred embodiment, the first apparatus is a protective cap unit. In this way, protection of the e.g. fragile second apparatus and/or fragile parts thereof may be obtained.

In a preferred embodiment, the one of said number of interrelated positions is obtained when said first apparatus is fitted onto said second apparatus.

In this way, exchange between the first and second apparatuses may be obtained very simply in connection with or more specifically after the use and storage of relevant data information by fitting the apparatuses to one another. This ensures in a very simple manner that the data information that needs to be exchanged is always exchanged after use and corresponding data generation, storage, etc. in at least one of the apparatuses. This may be obtained without the user having to perform any other action than following the normal procedure, e.g. fitting a protective cap unit onto the first apparatus after use.

Additionally, the energy/power used for communication between the apparatuses is minimized and/or reduced since only (very) short-range communication needs to be used. This is very important, especially for portable apparatuses, since a reduced energy consumption extends the time between the need for charging a power source of the apparatuses, like a battery, etc., prolongs the time where the apparatuses may be used and/or extends the life-time of a non-chargeable power source.

According to a preferred embodiment, the first apparatus comprises an integrated body fluid analyser. In this way, a relevant function of the self-treatment is always ready at hand for the patient.

In another embodiment, the first apparatus comprises an integrated wireless receiver for receiving data information from a third apparatus.

In yet another embodiment, the third apparatus is selected from the group of: a continuous blood glucose meter (CGM) located on the patient's body, a pulse monitor, a balance, and any other apparatus adapted to measure at least one physiological parameter.

Hereby relevant measurements may be obtained in a very easy fashion.

In yet another embodiment, the first apparatus is selected from the group of: a lipid monitor, a pulse monitor, a lancet device, a storage container, and a blood glucose monitor (BGM).

In another embodiment, the said second apparatus is a drug administration device.

In a preferred embodiment, the second apparatus is an insulin administration device.

According to another preferred embodiment, the data information is transmitted using one selected from the group of: optical communications means, inductive communications means, and electrical communications means.

In this way very simple short-range communication may be obtained.

In accordance with an embodiment, the said first and said second operations are selected from the group of: injection of medication, measurement of a body fluid, administering a number carbohydrates, and performing a physical activity.

In an embodiment, the first and/or second apparatus comprises means for suggesting/presenting a number of operations relevant for said treatment, thus enabling and assisting the patient to be at least partly in compliance with a specified/predetermined regime.

In this way, an insecure patient may obtain guidance with respect to the self-treatment, thus enhancing the confidence of the patient and broadening the understanding of the self-treatment by the patient.

Preferably, the data information relates to one or more of the following: amount of medication, type of medication, body fluid concentration, time stamp, amount of food, measurement of physical activity, notification of appointment, inventory logistics, and body characteristics.

In an embodiment, the data information is transmitted to a central server connected to a packet-switched network.

The data information may e.g. be transmitted by establishing a connection with wireless access point to the packet-switched network like the Internet, a TCP/IP network, a virtual private network (VPN), etc. or a GSM, UMTS, GPRS network.

In a preferred embodiment, the data information at said central server is accessible by relatives or any other relevant third parties by establishing a connection between a computer and said server.

In this way, relatives of e.g. a young or elderly patient, etc. may obtain a greater ease at mind, since they may simply log on to the server and obtain information of how well the user's situation is, e.g. how well a patient complies with a specified medical regime, how the patient is feeling, and whether a patient has remembered to do certain operations, tasks, etc. at/before/after a given time, etc. Preferably, the server comprises means for processing the data information in order to more clearly or better visualize the data information or derive additional information on the basis of the stored data information for the third parties.

In another embodiment, the date information at said central server is automatically transmitted to relatives and/or any other relevant third parties by e-mail or by other message formats to computers and/or mobile communications terminals. In this way, the relevant third parties, relatives, etc. automatically receives information of how well the user's situation is.

According to another embodiment, the data information at said central server is used in connection with a clinical trial of a predetermined product.

The product may e.g. be a new/modified type of medication, a new drug administration device, a new measuring device, etc.

The collected data information is automatically transmitted to a central location either periodically or at the end of the medical trial.

In this way, the quality of the collected data information is greatly enhanced, since only the factual data is automatically registered. Additionally, the time needed for performing a trial of a new product, which previously comprised time to manually input the data information on the basis of the patient's manual records/log-books, is reduced. Even further a validity check of data may be provided. These advantages may give great economic savings, since the time before a new product may be put on the market may be reduced. Possible errors like typos, etc. are reduced/eliminated.

Additionally, a greater safety for patients is obtained, since they may be monitored continuously or very closely.

In another embodiment, the data information at said central server is used by a physician, care-team, etc. in order to determine when a consultation is needed.

In this way, appointments/consultations for patients does not have to made on a regular basis but only when they are needed, which saves time and expenses. The physician also obtains a better preparation for a consultation, since any relevant data information may be analysed by the physician in advance by obtaining any relevant data information via the central server.

Finally, the invention also relates to a system of the aforementioned kind, where
- said first apparatus comprises storage means for automatically storing at least first data information relevant to said first operation,
- said second apparatus comprises storage means for automatically storing at least second data information relevant to said second operation, and
- that said first and second apparatuses comprise short-range communications means for automatically transmitting data information relevant to at least one of said first and second operations between said first and second apparatuses when said apparatuses are mutually positioned in one of said number of interrelated positions.

In one embodiment, the storage means of each apparatus comprises a unique and individual apparatus identification number, and the storage means of at least one apparatus comprises information relating to pairing a first apparatus and a second apparatus by linking their individual and unique identification number.

In one embodiment, the first apparatus is a protective cap unit.

In one embodiment, one of said number of interrelated positions is obtained when said first apparatus is fitted onto said second apparatus.

In one embodiment, the first apparatus comprises an integrated body fluid analyser.

In one embodiment, the first apparatus comprises an integrated wireless receiver for receiving data information from a third apparatus.

In one embodiment, the third apparatus is selected from the group of: a continuous blood glucose meter (CGM) located on the patient's body, a pulse monitor, a balance, and any other apparatus adapted to measure at least one physiological parameter.

In one embodiment, the first apparatus is selected from the group of: a lipid monitor, a pulse monitor, a lancet device, a storage container, and a blood glucose monitor (BGM).

In one embodiment, the second apparatus is a drug administration device.

In one embodiment, the second apparatus is an insulin administration device.

In one embodiment, the data information is transmitted using one selected from the group of: optical communications means, inductive communications means, and electrical communications means.

In one embodiment, the first and said second operations are selected from the group of: injection of medication, measurement of a body fluid, administering a number carbohydrates, and performing a physical activity.

In one embodiment, the first and/or second apparatus comprises means for suggesting/presenting a number of operations relevant for said treatment and assisting the patient to be at least partly in compliance with a specified/predetermined regime.

In one embodiment, the data information relates to one or more of the following: amount of medication, type of medication, body fluid concentration, time stamp, amount of food, measurement of physical activity, notification of appointment, inventory logistics, and body characteristics.

In one embodiment, the data information is transmitted to at least one selected from the group of a central server connected to a packet-switched network, a mobile telephone, a client connected to a packet-switched network, and another device capable of receiving, showing and/or processing the received data information.

In one embodiment, the information at said central server is accessible by relatives or any other relevant third parties by establishing a connection between a computer and said server.

In one embodiment, the date information at said central server is automatically transmitted to relatives and/or any other relevant third parties by e-mail or by other message formats to computers and/or mobile communications terminals.

In one embodiment, the data information at said central server is used in connection with a clinical trial of a predetermined product.

In one embodiment, the data information at said central server is used by a physician in order to determine when a consultation is needed.

The system and embodiments thereof correspond to the method and embodiments thereof and have the same advantages for the same reasons, and therefore will not be described again.

The present invention will now be described more fully with reference to the drawings, in which
Figure 1a illustrates two portable apparatuses according to the present invention;
Figure 1b illustrates a schematic cross-sectional view of a first apparatus and a second apparatus according to the present invention;
Figures 2a - 2c illustrate examples of various embodiments of the short-range communication means;
Figure 3 shows a first apparatus and second apparatus according to an alternative embodiment of the present invention;
Figure 4 illustrates a flow chart of a preferred embodiment according to the present invention;
Figure 5 illustrates the communication between a system of apparatuses and a central system;
Figure 6 shows a schematic block diagram of a first and a second apparatus according to the present invention.

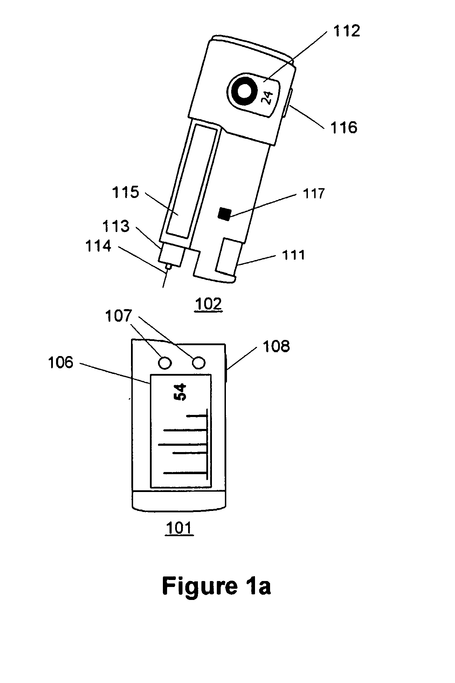

Figure 1a illustrates two portable apparatuses according to the present invention.

Shown are a first apparatus (101) and a second apparatus (102) for performing a first operation and a second operation, respectively, relevant for e.g. a medical self-treatment of and by a user.

The apparatuses (101; 102) have at least one interrelated position during normal use.

Each apparatus preferably has means for one or more of the following: storing, transmitting, receiving, processing and displaying data information.

The first apparatus automatically stores at least first data information relevant to the first operation during use by the patient, and the second apparatus automatically stores at least second data information relevant to the second operation during use.

During the normal use of the apparatuses in connection with the medical self-treatment, data information relevant to at least one of the first and second operations is automatically transmitted between the first and second apparatuses when the apparatuses are mutually positioned in one of a number of interrelated positions.

Preferably, one of the interrelated positions during which communication is initiated is when the first apparatus is fitted onto the second apparatus. In this way, the energy/power used for communication between the apparatuses is minimized and/or reduced since only (very) short-range communication needs to be used. This is very important, especially for portable apparatuses, since a reduced energy consumption extends the time between the need for charging a power source of the apparatuses, like a battery, etc., prolongs the time where the apparatuses may be used and/or extends the life-time of a non-chargeable power source.

In a preferred embodiment, the first apparatus (101) is a protective cap unit comprising an integrated blood glucose monitor (BGM), and the second apparatus (102) is an insulin administration device arranged so that they automatically transmit, via short-range communications means, data information relevant to at least one of the first and second operations between the first and second apparatuses when the apparatuses are mutually positioned in one of the number of interrelated positions, e.g. when the cap unit (101) is fitted to or docked with the medication administration device (102).

Alternatively, the second apparatus may be another type of drug administration device like a pen, syringe, inhaler, tablet dispenser, etc. or in general any medication administration device.

In this way, simplicity for the patient is obtained, since the apparatuses relevant for the self-treatment automatically store and exchange data information as part of the normal use.

Additionally, by using short-range communications means arranged to communicate in an interrelated position relevant for the normal use, a very simple, efficient, and relatively inexpensive way of communicating may be obtained, since short-range communications means may be of a simplified design.

In this way, the patient does not have to worry about collecting data information in a separate log-book and additionally, the data information may be collected in a single apparatus for further processing and/or use. In this way, a complete log-book is obtained in e.g. a single apparatus, which may be used by the patient with the help of the apparatuses to obtain detailed information of trends, current and/or previous state(s), re-occurring events, e.g. that adverse effects relating to the self-treatment occur every Sunday by using/analysing for behavioural and/or measured physiological patterns.

This also enhances the data quality of the data information and minimizes/eliminates the possibility of human error.

The doser (102) comprises input means/a user interface (111) e.g. a turning/adjusting wheel, a button, etc. for adjusting, either electronically or manually, the level/amount of medication to be administered, activation/input means (116) like a button, switch, etc. for initiating the administration of medication and a display (112) that shows the currently selected amount of medication to be administered with text, icons, graphic representations, etc. and other relevant and/or derived information. The doser (102) has processing means and storage facilities, like a CPU and RAM, for processing and automatically storing data, like the time, date and amount of medication that has been administered during use. That is, when a dose of medication is administered, the doser (102) stores the relevant information (like dose, type of medication, data and/or time of administration, etc.) in a memory (not shown) e.g. comprising a number of earlier automatically stored sets of data information. In this way, the patient does not have to perform any action in order to obtain a complete logbook of activities regarding the administration of medication.

The data information can be shown in the display (112) e.g. automatically and/or on request by the patient.

The doser (102) further comprises a cartridge (113) that contains the medication to be administered, and is fitted with a needle, syringe, etc. (114) through which the medication is administered. The doser (102) preferably has a transparent window (115) so that the amount of medication left in the cartridge (113) can readily be identified.

Cartridges (113) may contain different types of insulin, like fast acting, slow acting insulin, mix preparation, e.g. a 30/70 mix, etc., and the patient may insert/exchange a cartridge (113) of a given type when needed.

The first apparatus (102) is preferably provided with short-range communications means (117) for receiving and transmitting information and/or data representations from and to the first apparatus.

The first apparatus (101) is preferably a protective cap unit comprising an integrated BGM (101), which has receiving means (108) like a slot, opening, etc. for inserting test strips (not shown) containing a sample of blood, for analysis by the BGM (101) by operating suitable input means, user interface (UI), etc. like the buttons (107).

The BGM (101) has processing means and storage facilities, like a CPU and RAM, for processing and automatically storing data, like the time, date and measured blood glucose level (BGL). The result of the analysis/measurement is automatically stored in appropriate memory means (not shown) and e.g. shown in a display (106). The patient can at the same time be presented with the last couple of results over a time period e.g. also shown in the display (106) in the form of a graph bar, raw data, number values, etc. In this way, relevant BGL measurement are automatically obtained over time and kept in a sort of electronic log-book in the BGM (101).

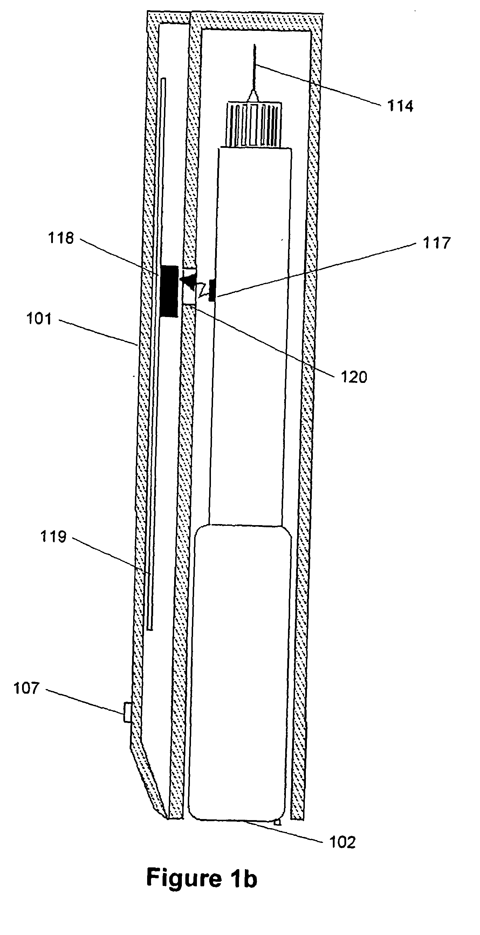

The cap/BGM (101) also comprises a short-range communications means (not shown) located on the inside of the cap/BGM (101), which is explained in greater detail in connection with Figure 1b showing a schematic cross-sectional view of a doser (102) and a cap unit/BGM (101) according to the present invention.

Alternatively, a pen, syringe, inhaler, etc. or in general any medication administration device may be used instead of a doser.

Alternatively, the first apparatus (101) comprises one or more of a body fluid analyser, a lipid monitor, a pulse monitor, a lancet device, and a storage container.

Figure 1b illustrates a schematic cross-sectional view of a first apparatus and a second apparatus according to the present invention.

Shown is a first apparatus/a cap unit (101) comprising an integrated BGM and a second apparatus/a doser (102), which correspond to the ones shown and explained in connection with Figure 1a.

The doser (102) and BGM (101) are shown mutually positioned in the preferred one of a number of interrelated positions for exchanging data information according to the invention. The interrelated position is when the cap/BGM (101) are fitted protectively onto the doser (102) after use, where either or both may have generated relevant data information. That is, the exchange of data information is initiated when the cap/BGM (101) are fitted onto the doser (102). Alternatively, the exchange of data information may be initiated when the first apparatus (101) is docked with the second apparatus (102).

In the shown interrelated position, the short-range communications means (117) of the doser (102) initiates communication with the short-range communications means (118) of the cap unit/BGM (101) or vice versa e.g. through a recess (120) or the like (enabling a clear/better communication path), thereby enabling a data information exchange between the two apparatuses (101; 102).

A sensor, switch, etc. may be used in order to determine when the cap (101) is fitted onto the doser (102). Alternatively, one of the apparatuses (101; 102) may perform polling in order to determine when communication may be initiated.

The short-range communications means (118) of the cap unit/BGM (101) is electronically connected/mounted on a control, processing and/or functional means (119), like a printed circuit board (PCB), of the BGM (101).

The short-range communications means (117; 118) is preferably an infrared (IR) communications means providing IR communication of data information between the first/BGM apparatus (101) and the second/doser apparatus (102).

Alternatively, the short-range communications means (117; 118) is an inductive means i.e. comprising inductive coils or the like in each apparatus.

As another alternative, the short-range communications (117; 118) is a electrical communications means, i.e. a simple switch mechanism that may be used to transfer data information between the apparatuses (101; 102).

Addtionally, the energy/power used for communication between the apparatuses is minimized and/or reduced since only (very) short-range communication needs to be used when the cap/BGM unit (101) is fitted on to the doser (102). This is very important, especially for portable apparatuses, since a reduced energy consumption extends the time between the need for charging a power source of the apparatuses, like a battery, etc., prolongs the time where the apparatuses may be used and/or extends the life-time of a non-chargeable power source.

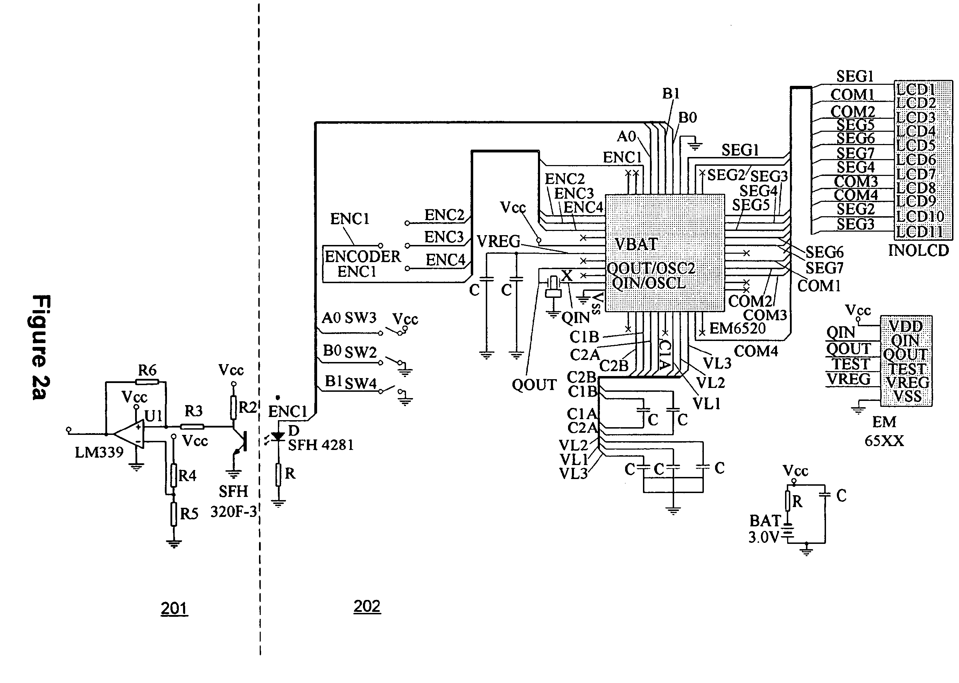

Figures 2a - 2c illustrate examples of various embodiments of the short-range communication means.

Figure 2a illustrates an embodiment of the short-range communication means adapted to communicate optically. Shown is an example of an embodiment of infrared (IR) communication means/transceivers. Shown is a receiver part (201) of a first apparatus and a transmitter part (202) of a second apparatus. Alternatively, the first apparatus and the second apparatus is each provided with a receiver (201) and a transmitter (202) thereby enabling two-way communication.

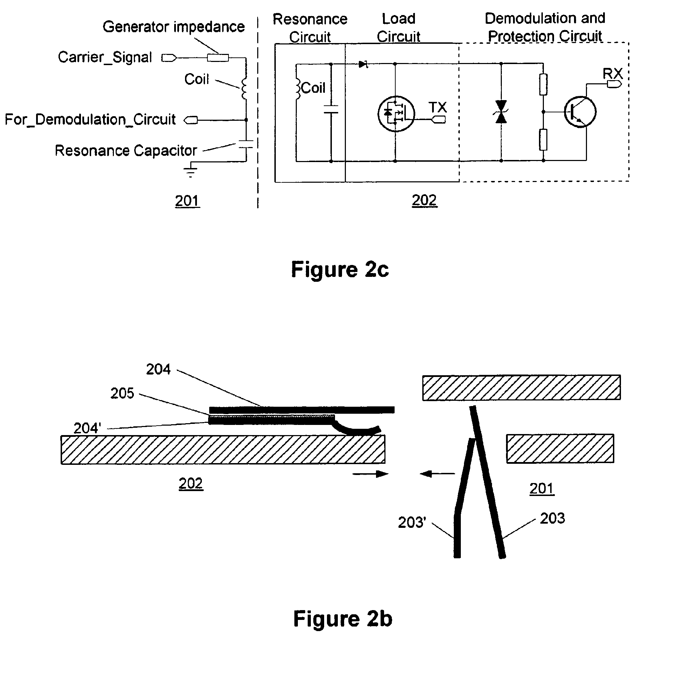

Figure 2b illustrates an embodiment of the short-range communication means adapted to communicate via a electrical switch. Shown is a cross-sectional view of an example of an embodiment of simple mechanical/electrical communication means in the form of switches. Shown are the communication switches of a first apparatus (201) and of a second apparatus (202). The communications switches (203; 203') of the first apparatus (201) have an electric connection between them when the first and second (201; 202) apparatus is not docked, fitted onto, in an interrelated communication position, etc. When the two apparatuses (201; 202) are brought together/in a interrelated position then a first switch/switch part (204) of the second apparatus (202) touches and moves the first switch/switch part (203) of the first apparatus (201) thereby establishing an electronic connection between them (203, 204) and breaking the connection of switch/switch part (203) and (203'). During the same movement a second switch/switch part (204') of the second apparatus (202) touches the second switch/switch part (203') of the first apparatus (201) thereby establishing an electronic connection. The breaking of the connection between the first switch/switch part (203) and the second switch/switch part (203') may determine when communication, transfer of information, etc. may be initiated.

The first (204) and second switch/switch part (204') of the second apparatus is preferably separated by an insulation layer (205).

Figure 2c illustrates an embodiment of the short-range communication means adapted to communicate via inductive communication. Shown is an example of an embodiment of simple inductive communication means where current induced in a resonance circuit is used to transfer information. Shown is a receiver part (201) of a first apparatus and a transmitter part (202) of a second apparatus. Alternatively, the first apparatus and the second apparatus is each provided with a receiver (201) and a transmitter (202) thereby enabling two-way communication.

The shown examples all enable communication in a very simple fashion with minimal energy used.

Alternatively, other types of communication may be used like RF-communication, e.g. Bluetooth or other types, etc.

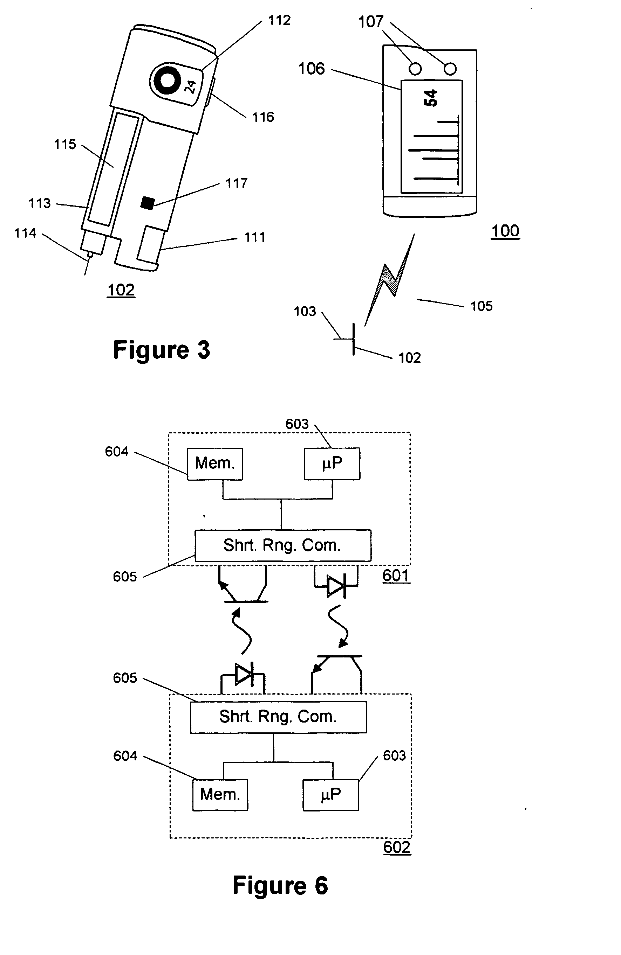

Figure 3 shows a first apparatus and second apparatus according to an alternative embodiment of the present invention.

Shown is a second apparatus/a doser (102), which may correspond to the doser shown in and explained in connection with Figures 1a and 1b.

Additionally, a first apparatus is shown in the form of a protective cap unit comprising a continuous glucose monitor (CGM) (100).

The CGM (100) is an apparatus that monitors/measures the blood glucose level/concentration of a patient continuously or at least at a regular interval and, in this embodiment, comprises a base unit (100) and a glucose biosensor (103).

In this embodiment, the CGM base unit (100) is connected via wireless communications means (not shown) like an RF transceiver, etc. to the biosensor (103). Alternatively, the base unit (100) and biosensor (103) may be electronically connected via a wire or the like.

The glucose biosensor (103) is mounted on an adhesive (102) located on an appropriate part of the patient's body, like the stomach, upper arm, etc. and is located subcutaneously, i.e. in the external fat, in the patient's body.

The biosensor (103) preferably comprises a potentiostat where a fixed potential can be applied between two electrodes of the biosensor, thereby measuring the current that the work electrode of the biosensor produces. The generated current is proportional to the glucose concentration in the blood of the patient.

A signal representing the generated current is sent via the wireless connection (105) or a wire to the CGM base unit (100) for storage, presentation, etc. The translation/interpretation from a continuous signal into a representation for later processing is preferably performed by a standard A/D converter with a sampling rate which at least is faster than the worst case change of the BGL, so that even the fastest change is 'captured' by the CGM/CGM base unit (100). Alternatively, a value corresponding to a number of averaged/integrated samples over a period of e.g. a couple of seconds, minutes, etc. may be transmitted via the wireless connection (105) or the wire. Alternatively, the continuous signal may be transmitted directly to the base unit (100).

The converted measurement/continuous value may be presented to the patient via displaying means (106), like an LCD display, a (graphical) user interface ((G)UI), etc.

The converted measurement is preferably also stored automatically in a suitable memory (not shown) in the CGM base unit (100) and may in this way be kept for later retrieval, analysis, processingetc., so that a detailed history log of sampled measurements may be obtained. This detailed history log may e.g. be used to predict a trend for the BGL of a patient, thereby enhancing the information value for the patient.

The CGM base unit (100) preferably also comprises short-range communications means (not shown) for communicating in a simple fashion during normal use with the communications means (117) on the doser (102) for receiving and/or transmitting the automatically stored data information, as described in connection with Figures 1a and 1b.

In an embodiment of the present invention the BGL measurement is converted into a corresponding amount of insulin needed to bring the patient into compliance and displayed on the display (106).

The biosensor (103) is preferably calibrated on a regular basis, e.g. each day, by external calibration e.g. by a traditional blood glucose monitor (BGM) system, in order to ensure the best accuracy. Typically, the biosensor (103) will have to be replaced after e.g. three days of use and be calibrated once each day.

Alternatively, other invasive, semi-invasive or non-invasive types of systems may embody the CGM (100).

The CGM (100) may also be provided with communications means (not shown) for receiving and transmitting information and data representation from and to other apparatuses. Alternatively, the means for communicating with the biosensor (103) may be used.

In a preferred embodiment one of the apparatuses, e.g. the CGM unit (100), comprises processing/calculation means and storage means (not shown) that estimate an expected change for the blood glucose level in order to derive a trend analysis of the BGL. This estimation may be obtained on the basis of the previously measured blood glucose levels, amount of administered medication, time and date stamp, values of measured and/or inputted physiological parameters, etc.

Additionally, one of the apparatuses e.g. the CGM (100) may also comprise means for giving a notification, warning, etc., e.g. by sound, blinking text/graphic and the like, if the blood glucose level and/or the prediction of the blood glucose level drops outside a predetermined interval, e.g. 4 to 6 mMolar (mM) glucose in order to attract the patient's attention to a potentially inappropriate/dangerous situation, so that the patient can initiate the proper steps to manage the situation.

The use of the apparatuses/system may e.g. be illustrated by the following examples.

### Situation 1.

A diabetic is about to eat a meal. The diabetic observes that the glucose value is normal (5 mM) and has been so constantly for the last 60 minutes. The diabetic then knows by experience that it is necessary to inject e.g. 10 IU insulin because of the impending meal, in order to have a glucose value in the normal range again after the meal.

### Situation 2.

A diabetic is about to eat a meal. The diabetic observes that the glucose level is low (4 mM) and is additionally decreasing at a great rate. The diabetic then knows by experience that it is necessary to inject less insulin than usual, e.g. 6 IU insulin because of the impending meal, in order to have a glucose value in the normal range again after the meal.

### Situation 3

A diabetic is about to eat a meal. The diabetic observes that the glucose value is high (6 mM) and is additionally increasing at a great rate. The diabetic then knows by experience that it is necessary to inject more insulin than usual, e.g. 14 IU insulin because of the impending meal, in order to have a glucose value in the normal range again after the meal.

### Situation 4

A diabetic is about to take a long swim. The diabetic observes that the glucose value is normal (5 mM), but is decreasing, and therefore knows that it will be dangerous to start the swim, as muscular exertion increases the ability of the cells to use insulin and convert glucose. A small meal is therefore necessary before the swim in order not to get below the determined normal limit.

### Situation 5

A diabetic is about to go to bed. The diabetic observes that the glucose value is 6 and is increasing at a great rate. The diabetic then knows by experience that it is necessary to inject 6 IU slow-acting insulin in order to stay at a glucose level within the determined limits during the night.

The system enables a patient/user (with his help) to be in close compliance/metabolic control, thereby reducing the risk of diabetic acute and late complications.

Preferably, at least one apparatus of the system may comprise means for supplementing or making up for lack of the diabetic's experience in everyday situations like the 5 above by presenting choices to the patient as described in US Patent Application No. 09/462,128 incorporated herein by reference.

This may be obtained by enabling the system to present different choices to the patient, which fully and/or partly ensure a compliance situation for the patient if followed.

Hereby, the patient's self-treatments change from restrictions to possibilities, thereby enhancing the overall 'quality-of-life' for the user and better ensuring that the patient's self-treatment complies better or fully with a specified regimen by choosing proposed choices which comply with the regimen. This avoids the risk that the user chooses actions and alternatives that do not fully or at all correspond to the optimal regimen due to a lack of a clear overview of the complex factors involved in the self-treatment.

In order to present choices to the patient a prediction of how the patient's metabolism will react according to the proposed choices is needed. This may e.g. be provided on the basis of a dynamic model representing the human metabolism, as e.g. disclosed in US Patent Application No. 09/462,128 or in the general literature.

In a preferred embodiment, the data information is transmitted to a central server/computer system for storage, processing, etc., e.g. via a wireless access point (e.g. using Bluetooth) to a packet-switched/TCP-IP network like the Internet, a virtual private network (VPN), or UMTS, GSM, GPRS, etc. This may be done each time the first and second apparatuses exchange information via the short-range communications means, and preferably only new, non-replicated, i.e. not existing on the server, etc., data information is transmitted.

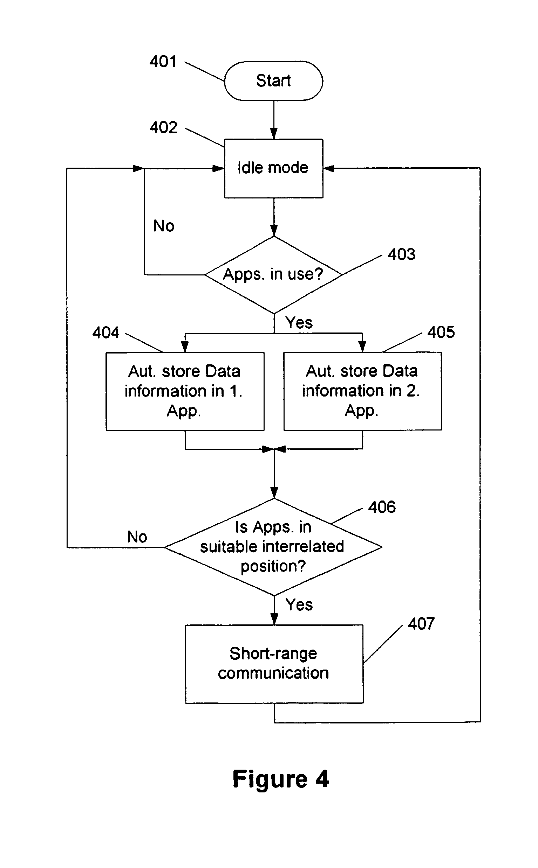

Figure 4 illustrates a flow chart of a preferred embodiment according to the present invention. The method starts in step (401).

In step (402) the apparatuses according to the present invention are in an idle mode/state.

A test in step (403) checks whether at least one of the apparatuses is being used, i.e. generates information. If that is not the case then the idle mode in step (402) is resumed/continued. If one of the apparatuses is being used and generates data, then the relevant information is automatically stored in the respective apparatus in step (404) and step (405). A check is made in step (406) whether the apparatuses are mutually positioned in a suitable interrelated position. If that is the case, step (407) is executed where a (very) short-range communication is initiated and executed automatically so that the information is transmitted from one apparatus to the other and/or vice versa. If the check in step (406) is 'No', the apparatuses return to idle mode in step (402).

Preferably, the check in step (406) is performed independently of the check in step (403), i.e. the data/information generation, storage, etc. are done independently of the short-range communication of any stored information from a second apparatus to a first apparatus or vice versa. The steps (406 and 403) and associated processes may be done in parallel or in an alternating fashion.

Preferably, the first apparatus is a protective cap unit comprising an integrated body fluid analyser. In this way, protection of the e.g. fragile second apparatus and/or fragile parts thereof may be obtained, and a relevant function of the self-treatment is always ready at hand for the patient.

Preferably, an interrelated position is obtained when the first apparatus is fitted onto or docked with the second apparatus.

In this way, exchange between the first and second apparatuses may be obtained very simply in connection with or more specifically after the use and storage of relevant data information by fitting the apparatuses to one another. This ensures in a very simple manner that the data information that needs to be exchanged is always exchanged after use and corresponding data generation, storage, etc. in at least one of the apparatuses. This may be obtained without the user having to perform any other action than following the normal procedure, e.g. fitting a protective cap unit onto the first apparatus after use.

According to a preferred embodiment, the step (407) of automatically transmitting data information between the first and second apparatuses comprises checking for an unique and individual apparatus identification number for each apparatus, and pairing a first apparatus and a second apparatus by linking their individual and unique identification numbers.

In this way a given first apparatus and at least a given second apparatus may be paired to form a single corresponding set. A given apparatus may check whether a communicating apparatus is a paired one and only initiate communication in the affirmative. Hereby, a given first apparatus may only exchange data information with at least a second apparatus e.g. belonging to the same user, the patient's physician or any other appropriate and approved apparatuses.

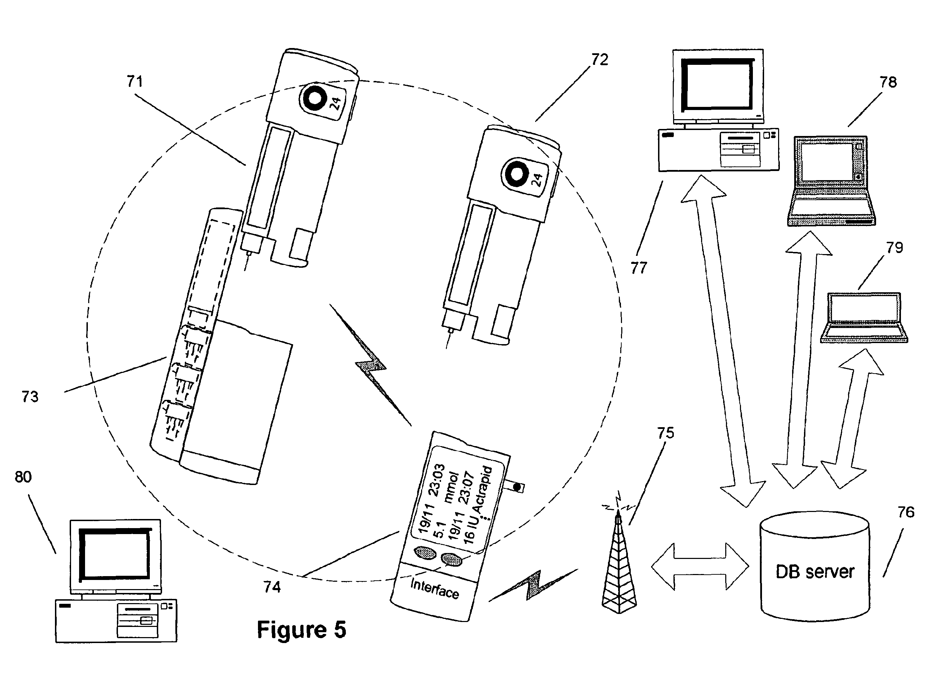

Figure 5 illustrates the communication between a system of apparatuses and a central system. Shown are a first (74) and a second apparatus (72) according to the present invention. Additionally, another apparatus (71) is shown that may communicate wirelessly with the first apparatus (74) or may be placed in an interrelated communication position with the first apparatus (74). The other apparatus (71) may e.g. contain fast acting insulin while the second apparatus (72) may e.g. contain slow acting insulin or a mix preparation.

The drug administration devices (71, 72) comprises a micro controller and memory. The devices (71, 72) are capable of holding information about the insulin type they contain. This information may either be obtained by the relevant apparatus reading e.g. a bar code on the cartridge containing the drug or the information may be specified by the user. Thus the features of each device (71, 72) enable it to log information about the insulin treatment (insulin type, size of administered doses and corresponding time/date stamp).

One apparatus (71) may e.g. be equipped with a cap unit (73) which acts as a storage container for an extra insulin cartridge, needles etc.

The second apparatus (72) is equipped with the first apparatus (74) in the shape of a cap unit comprising a integrated BGM, a micro controller and memory. This enables the cap unit/BGM (74) to log information about the measured blood glucose concentration (with time and date stamp).

In the present example the first apparatus (74) in addition to the short-range communication interface, comprises a communication interface that enables it to communicate with external units through standard communication links (RS-232, Wireless local area network, phone, cellular phone, pager, satellite link, etc.). Through these communication links, the patient's treatment data can be transferred to the patient's own computer (80) and/or via e.g. the telephone/mobile system (75) to the patient's electronic medical record on a central server (76). From here, the treatment data may be accessed by the patient e.g. from a web page, using a stationary computer (77), a laptop computer (78), a handheld computer (79), etc. Apart from the patient, the care-team can access the patient's treatment data. Additionally, the first apparatus (74) may receive data information from the central server (76) in addition to transmitting data.

In this way, each apparatus obtains and stores relevant data information and transmits the data information to the first apparatus (74) like described above. The information/treatment data may supply the patient with an overview of his treatment and present choices as well as warnings or alarms if data shows that a potential dangerous situation may occur.

When the first apparatus (74) is connected to the central server (76) through standard communication links, the treatment data is transferred to the patient's electronic medical record. This enables an expert system on the central server to notify the care-team if needed. The care-team may send information back to the user or send help if needed.

The information at the server (76) may e.g. be accessible/obtainable by relatives or any other relevant third parties by establishing a connection between a computer and the server e.g. using a browser. A parent/a relative may also receive an e-mail, a SMS message or another message in a suitable format containing information regarding a child, elderly person's, etc. actual condition (e.g. blood glucose measurement/reading and time/date stamp) and time, amount, etc. for last administration of medication as well as other relevant (e.g. processed, derived) information like a trend, warnings, etc. This information may also be sent directly to the parent/relative from the first apparatus (74) via a telephone/mobile communication system (75). In this way parents and other relatives is assured that everything is ok with a child, an elderly, another relative, etc. and may take action otherwise.

The information at the server (76) may also be used in connection with a clinical trial of a predetermined product, like described above.

Additionally, the information at the central server (76) may be used by a physician, professional, etc. in order to determine when a consultation is needed. This saves time for the physician, professional, etc. and the user/patient since a consultation is only arranged when there is a need. The physician will also have the treatment data/information ready at hand prior to a consultation instead of receiving it during the consultation, which enables the physician to be better prepared. Additionally, the close monitoring of the user/patient also enables the physician, professional, etc. to act much quicker if a potentially dangerous situation arises.

Figure 6 shows a schematic block diagram of a first and a second apparatus according to the present invention. Shown is a first apparatus (601) and a second apparatus (602) each comprising one or more microprocessor units (603), a memory (604) and short-range communication means (605) in this example in the form of two-way IR communication transceivers and receivers. The memory/storage (604) may comprise non-volatile memory, volatile memory, or both. Additionally, each apparatus (601; 602) may comprise means of one or more of the following a display, UI, GUI, a BGM, CGM, communication means for communicating with external devices (like Bluetooth, etc.), medication delivery, etc. as described elsewhere.

## Claims

1. A method of controlling data information between two portable medical apparatuses (101; 102), the use of the apparatuses including a first operation and a
second operation, said portable apparatuses comprising a first apparatus (101) for
performing the first operation and a second apparatus (102) for performing the
second operation, where each apparatus has means for one or more of the
following: storing, transmitting, receiving, processing and displaying data information, and where the two apparatuses have a number of interrelated
positions during normal use, **characterized in that** the method comprises the steps of:
• automatically storing at least first data information relevant to said fi rst operation in said first apparatus (101),
• automatically storing at least second data information relevant to said second operation in said second apparatus (102), and
• automatically transmitting, via short-range communications means (117; 118), data information relevant to at least one of said first and second operations between said first and second apparatuses when said apparatuses are mutually positioned in one of said number of interrelated positions,
wherein said second apparatus is a drug administration device.

2. A method according to claim 1, **characterized in that** the step of automatically transmitting data information between said first and second apparatuses comprises one or more of the following:
• checking a unique and individual apparatus identification number for each apparatus, and
• pairing a first apparatus and a second apparatus by linking their individual and unique identification numbers.

3. A method according to claims 1 - 2, **characterized in that** said first apparatus is a protective cap unit.

4. A method according to claims 1 - 3, **characterized in that** one of said number of interrelated positions is obtained when said first apparatus is fitted onto said second apparatus.

5. A method according to claims 1 - 4, **characterized in that** said first apparatus comprises an integrated body fluid analyser.

6. A method according to claims 1 - 5, **characterized in that** said first apparatus comprises an integrated wireless receiver for receiving data information from a third apparatus.

7. A method according to claim 6, **characterized in that** said third apparatus is selected from the group of:
• a continuous blood glucose meter (CGM) located on the patient's body,
• a pulse monitor,
• a balance,
• and any other apparatus adapted to measure at least one physiological parameter.

8. A method according to claims 1 - 7, **characterized in that** said fi rst apparatus is selected from the group of:
• a lipid monitor,
• a pulse monitor,
• a lancet device,
• a storage container, and
• a blood glucose monitor (BGM).

9. A method according to claims 1-8, **characterized in that** said second apparatus is an insulin administration device.

10. A method according to claims 1 - 9, **characterized in that** said data information is transmitted using one selected from the group of:
• optical communications means,
• inductive communications means, and
• electrical communications means.

11. A method according to claims 1-10, **characterized in that** said first and second operations are selected from the group of:
• injection of medication,
• measurement of a body fluid,
• administering a number of carbohydrates, and
• performing a physical activity.

12. A method according to claims 1 - 11, **characterized in that** said first and/or said second apparatus comprises means for suggesting and/or presenting a number of operations relevant for said treatment and assisting the patient to be at least partly in compliance with a specified/predetermined regime.

13. A method according to claims 1-12, **characterized in that** said data information relates to one or more of the following:
• amount of medication,
• type of medication,
• body fluid concentration,
• time stamp,
• amount of food,
• measurement of physical activity,
• notification of appointment,
• inventory logistics, and
• body characteristics.

14. A method according to claims 1-13, **characterized in that** said data information is transmitted to at least one selected from the group of
• a central server (76) connected to a packet-switched network,
• a mobile telephone,
• a client connected to a packet-switched network, and
• another device capable of receiving, showing and/or processing the received data information.

15. A method according to claim 14, **characterized in that** said data
information at said central server is accessible by relatives or any other relevant third parties by establishing a connection between a computer (77; 78; 79) and said server.

16. A method according to claims 14-15, **characterized in that** said
data information at said central server is automatically transmitted to relatives
and/or any other relevant third parties by e-mail or by other message formats
to computers and/or mobile communications terminals.

17. A method according to claims 14-16, **characterized in that** said
data information at said central server is used in connection with a clinical trial of a predetermined product.

18. A method according to claims 14-17, **characterized in that** said
data information at said central server is used by a physician in order to determine when a consultation is needed.

19. A system for controlling data information between two portable apparatuses (101; 102), the use of the apparatuses including a first operation and a
second operation, said portable apparatuses comprising a first apparatus (101) for
performing the first operation and a second apparatus (102) for performing the second operation, where each apparatus has means for one or more of the following: storing, transmitting, receiving, processing and displaying data information, and where the two apparatuses have a number of interrelated
positions during normal use, **characterized in that**
• said first apparatus comprises storage means for automatically storing at least first data information relevant to said first operation.
• said second apparatus comprises storage means for automatically storing at least second data information relevant to said second operation, and
• that said first and second apparatuses comprise short-range communications means (117; 118) for automatically transmitting data information relevant to at least one of said first and second operations between said first and second apparatuses when said apparatuses are mutually positioned in one of said number of interrelated positions,
wherein said second apparatus is a drug administration device.

20. System according to claim 19. **characterized in that** the storage
means of each apparatus comprises a unique and individual apparatus identification number and that the storage means of at least one apparatus
comprises information relating to pairing a first apparatus and a second apparatus by linking their individual and unique identification numbers.

21. System according to claims 19-20, **characterized in that** said first apparatus is a protective cap unit.

22. System according to claims 19 - 21, **characterized in that** one of said number of interrelated positions is obtained when said first apparatus is fitted onto said second apparatus.

23. System according to claims 19 - 22, **characterized in that** said first apparatus comprises an integrated body fluid analyser.

24. System according to claims 19 - 23, **characterized in that** said first apparatus comprises an integrated wireless receiver for receiving data information from a third apparatus.

25. System according to claim 24, **characterized in that** said third apparatus is selected from the group of:
• a continuous blood glucose meter (CGM) located on the patient's body,
• a pulse monitor,
• a balance,
• and any other apparatus adapted to measure at least one physiological parameter.

26. System according to claims 19 - 25, **characterized in that** said first apparatus is selected from the group of:
• a lipid monitor,
• a pulse monitor,
• a lancet device,
• a storage container, and
• a blood glucose monitor (BGM).

27. System according to claims 19 - 26, **characterized in that** said second apparatus is an insulin administration device.

28. System according to claims 19 - 27, **ccharacterized in that** said data information is transmitted using one selected from the group of:
• optical communications means,
• inductive communications means, and
• electrical communications means.

29. System according to claims 19 - 28, **characterized in that** said first and second operations are selected from the group of:
• injection of medication,
• measurement of a body fluid,
• administering a number of carbohydrates, and
• performing a physical activity.

30. System according to claims 19 - 29, **characterized in that** said first and/or said second apparatus comprises means for
suggesting/presenting a number of operations relevant for said treatment and
assisting the patient to be at least partly in compliance with a specified/predetermined regime.

31. System according to claims 19 - 30, **characterized in that** said data information relates to one or more of the following:
• amount of medication,
• type of medication,
• body fluid concentration,
• time stamp,
• amount of food,
• measurement of physical activity,
• notification of appointment,
• inventory logistics, and
• body characteristics.

32. System according to claims 19 - 31, **characterized in that** said data information is transmitted to at least one selected from the group of
• a central server (76) connected to a packet-switched network,
• a mobile telephone,
• a client connected to a packet-switched network, and
• another device capable of receiving, showing and/or processing the received data information.

33. System according to claim 32, **characterized in that** said data information at said central server is accessible by relatives or any other relevant third parties by establishing a connection between a computer (77; 78; 79) and said server.

34. A method according to claim 32 - 33, **characterized in that** said
data information at said central server is automatically transmitted to relatives
and/or any other relevant third parties by e-mail or by other message formats
to computers and/or mobile communications terminals.

35. System according to claim 32 - 34, **characterized in that** said data information at said central server is used in connection with a clinical trial of a predetermined product.

36. System according to claims 32 - 35, **characterized in that** said data information at said central server is used by a physician in order to determine when a consultation is needed.

## Patentansprüche

1. Verfahren zum Steuern von Dateninformationen zwischen zwei tragbaren medizinischen Geräten (101; 102), wobei die Verwendung der Geräte einen ersten Vorgang und einen zweiten Vorgang einschließt, wobei die tragbaren Geräte ein erstes Gerät (101) zum Durchführen des ersten Vorgangs und ein zweites Gerät (102) zum Durchführen des zweiten Vorgangs umfasst, wobei jedes Gerät Mittel für eines oder mehrere von Folgendem aufweist: Speichern, Senden, Empfangen, Verarbeiten und Anzeigen von Dateninformationen, und wobei die beiden Geräte während normaler Verwendung eine Anzahl an in Wechselbeziehung stehenden Positionen aufweisen, **dadurch gekennzeichnet, dass** das Verfahren die folgenden Schritte umfasst:
- automatisches Speichern mindestens einer für den ersten Vorgang im ersten Gerät (101) relevanten ersten Dateninformation,
- automatisches Speichern mindestens einer für den ersten Vorgang im zweiten Gerät (102) relevanten zweiten Dateninformation und
- automatisches Senden über Kurzstreckenkommunikationsmittel (117; 118) von
für mindestens einen des ersten und des zweiten Vorgangs relevanten Dateninformationen zwischen dem ersten und dem zweiten Gerät, wenn die Geräte in der Anzahl von in Wechselbeziehung stehenden Positionen gegenseitig positioniert sind,
wobei das zweite Gerät eine Arzneistoffverabreichungsvorrichtung ist.

2. Verfahren nach Anspruch 1, **dadurch gekennzeichnet, dass** der Schritt des automatischen Sendens von Dateninformationen zwischen dem ersten und dem zweiten Gerät eines oder mehrere von Folgendem umfasst:
- Überprüfen einer einzigartigen und individuellen Geräteidentifikationsnummer für jedes Gerät und
- Paarung eines ersten Geräts und eines zweiten Geräts durch Verknüpfen ihrer individuellen und einzigartigen Identifikationsnummern.

3. Verfahren nach den Ansprüchen 1-2, **dadurch gekennzeichnet, dass** das erste Gerät eine Schutzkappeneinheit ist.

4. Verfahren nach den Ansprüchen 1-3, **dadurch gekennzeichnet, dass** eine der Anzahl von in Wechselbeziehung stehenden Positionen erhalten wird, wenn das erste Gerät auf dem zweiten Gerät aufgesetzt ist.

5. Verfahren nach den Ansprüchen 1-4, **dadurch gekennzeichnet, dass** das erste Gerät einen eingebauten Körperfluidanalysator umfasst.

6. Verfahren nach den Ansprüchen 1-5, **dadurch gekennzeichnet, dass** das erste Gerät einen eingebauten drahtlosen Empfänger zum Empfangen von Dateninformationen von einem dritten Gerät umfasst.

7. Verfahren nach Anspruch 6, **dadurch gekennzeichnet, dass** das dritte Gerät ausgewählt ist aus der Gruppe:
- eines sich auf dem Körper des Patienten befindenden kontinuierlichen Blutglucosemessers (continuous blood glucose meter; CGM),
- eines Pulsüberwachungsgeräts,
- einer Waage
- und eines beliebigen anderen Geräts, das zum Messen mindestens eines physiologischen Parameters angepasst ist.

8. Verfahren nach den Ansprüchen 1-7, **dadurch gekennzeichnet, dass** das erste Gerät ausgewählt ist aus der Gruppe:
- eines Lipidüberwachungsgeräts,
- eines Pulsüberwachungsgeräts,
- einer Stechhilfe,
- eines Vorratsbehälters und
- eines Blutglucoseüberwachungsgeräts (blood glucose monitor; BGM).

9. Verfahren nach den Ansprüchen 1-8, **dadurch gekennzeichnet, dass** das zweite Gerät eine Insulinverabreichungsvorrichtung ist.

10. Verfahren nach den Ansprüchen 1-9, **dadurch gekennzeichnet, dass** die Dateninformationen unter Verwendung von einem gesendet wird, das ausgewählt ist aus der Gruppe:
- optischer Kommunikationsmittel,
- induktiver Kommunikationsmittel und
- elektrischer Kommunikationsmittel.

11. Verfahren nach den Ansprüchen 1-10, **dadurch gekennzeichnet, dass** der erste und der zweite Vorgang ausgewählt sind aus der Gruppe:
- Medikamenteninjektion,
- Messung eines Körperfluids,
- Verabreichen einer Anzahl von Kohlenhydraten und
- Durchführen einer physikalischen Aktivität.

12. Verfahren nach den Ansprüchen 1-11, **dadurch gekennzeichnet, dass** das erste und/oder das zweite Gerät Mittel zum Vorschlagen und/oder Darbieten einer Anzahl von Vorgängen umfasst, die für die Behandlung und Unterstützung des Patienten, zumindest teilweise eine spezifizierte/vorbestimmte Verordnung zu befolgen, relevant sind.

13. Verfahren nach den Ansprüchen 1-12, **dadurch gekennzeichnet, dass** die Dateninformationen eines oder mehrere von Folgendem betreffen:
- Medikamentenmenge,
- Medikamententyp,
- Körperfluidkonzentration,
- Zeitstempel,
- Nahrungsmenge,
- Messung von physikalischer Aktivität,
- Terminmitteilung,
- Bestandslogistik und
- Körpereigenschaften.

14. Verfahren nach den Ansprüchen 1-13, **dadurch gekennzeichnet, dass** die Dateninformationen zu mindestens einem gesendet werden, das ausgewählt ist aus der Gruppe:
- eines an ein paketvermitteltes Netzwerk angeschlossenen Zentralservers (76),
- eines Mobiltelefons,
- eines an ein paketvermitteltes Netzwerk angeschlossenen Clients und
- einer anderen Vorrichtung, die in der Lage ist, die empfangenen Dateninformationen zu empfangen, anzuzeigen und/oder zu verarbeiten.

15. Verfahren nach Anspruch 14, **dadurch gekennzeichnet, dass** die Dateninformationen am Zentralserver Angehörigen oder jeder beliebigen anderen relevanten dritten Partei zugänglich ist, indem eine Verbindung zwischen einem Computer (77; 78; 79) und dem Server aufgebaut wird.

16. Verfahren nach den Ansprüchen 14-15, **dadurch gekennzeichnet, dass** die Dateninformationen am Zentralserver automatisch an Angehörige und/oder jede beliebige relevante dritte Partei per E-Mail oder durch andere Nachrichtenformate an Computer und/oder Mobilkommunikationsendgeräte gesandt werden.

17. Verfahren nach den Ansprüchen 14-16, **dadurch gekennzeichnet, dass** die Dateninformationen am Zentralserver zusammen mit einer klinischen Studie eines vorbestimmten Produkts verwendet werden.

18. Verfahren nach den Ansprüchen 14-17, **dadurch gekennzeichnet, dass** die Dateninformationen am Zentralserver von einem Arzt verwendet werden, um zu ermitteln, wann eine Konsultation erforderlich ist.

19. System zum Steuern von Dateninformationen zwischen zwei tragbaren Geräten (101; 102), wobei die Verwendung der Geräte einen ersten Vorgang und einen zweiten Vorgang umfasst, wobei die tragbaren Geräte ein erstes Gerät (101) zum Durchführen des ersten Vorgangs und ein zweites Gerät (102) zum Durchführen des zweiten Vorgangs einschließt, wobei jedes Gerät Mittel für eines oder mehrere von Folgendem aufweist: Speichern, Senden, Empfangen, Verarbeiten und Anzeigen von Dateninformationen, und wobei die beiden Geräte eine Anzahl an in Wechselbeziehung stehenden Positionen während normaler Verwendung aufweisen, **dadurch gekennzeichnet, dass**
- das erste Gerät Speichermittel zum automatischen Speichern zumindest der für den ersten Vorgang relevanten ersten Dateninformation umfasst,
- das zweite Gerät Speichermittel zum automatischen Speichern zumindest der für den zweiten Vorgang relevanten zweiten Dateninformation umfasst,
- das erste und das zweite gerät Kurzstreckenkommunikationsmittel (117; 118) zum automatischen Senden von für den ersten und den zweiten Vorgang relevanten Dateninformationen zwischen dem ersten und dem zweiten Gerät, wenn die Geräte in der Anzahl von in Wechselbeziehung stehenden Positionen gegenseitig positioniert sind, umfasst,
wobei das zweite Gerät eine Arzneistoffverabreichungsvorrichtung ist.

20. System nach Anspruch 19, **dadurch gekennzeichnet, dass** das Speichermittel jedes Gerät eine einzigartige und individuelle Geräteidentifikationsnummer umfasst und dass das Vorratsmittel mindestens eines Geräts Informationen umfasst, die die Paarung eines ersten Geräts und eines zweiten Geräts durch Verknüpfen ihrer individuellen und einzigartigen Identifikationsnummern betreffen.

21. System nach den Ansprüchen 19-20, **dadurch gekennzeichnet, dass** das erste Gerät eine Schutzkappeneinheit ist.

22. System nach den Ansprüchen 19-21, **dadurch gekennzeichnet, dass** eine der Anzahl von in Wechselbeziehung stehenden Positionen erhalten wird, wenn das erste Gerät auf das zweite Gerät aufgesetzt ist.

23. System nach den Ansprüchen 19-22, **dadurch gekennzeichnet, dass** das erste Gerät einen eingebauten Körperfluidanalysator umfasst.

24. System nach den Ansprüchen 19-23, **dadurch gekennzeichnet, dass** das erste Gerät einen eingebauten drahtlosen Empfänger zum Empfangen von Dateninformationen von einem dritten Gerät umfasst.

25. System nach Anspruch 24, **dadurch gekennzeichnet, dass** das dritte Gerät ausgewählt ist aus der Gruppe:
- eines sich auf dem Körper des Patienten befindenden kontinuierlichen Blutglucosemessers (CGM),
- eines Pulsüberwachungsgeräts,
- einer Waage
- und eines beliebigen anderen Geräts, das zum Messen mindestens eines physiologischen Parameters angepasst ist.

26. System nach den Ansprüchen 19-25, **dadurch gekennzeichnet, dass** das erste Gerät ausgewählt ist aus der Gruppe:
- eines Lipidüberwachungsgeräts,
- eines Pulsüberwachungsgeräts,
- einer Stechhilfe,
- eines Vorratsbehälters und
- eines Blutglucoseüberwachungsgeräts (BGM).

27. System nach den Ansprüchen 19-26, **dadurch gekennzeichnet, dass** das zweite Gerät eine Insulinverabreichungsvorrichtung ist.

28. System nach den Ansprüchen 19-27, **dadurch gekennzeichnet, dass** die Dateninformationen unter Verwendung von einem gesendet wird, das ausgewählt ist aus der Gruppe:
- optischer Kommunikationsmittel,
- induktiver Kommunikationsmittel und
- elektrischer Kommunikationsmittel.

29. System nach den Ansprüchen 19-28, **dadurch gekennzeichnet, dass** der erste und der zweite Vorgang ausgewählt sind aus der Gruppe:
- Medikamenteninjektion,
- Messung eines Körperfluids,
- Verabreichen einer Anzahl von Kohlenhydraten und
- Durchführen einer physikalischen Aktivität.

30. System nach den Ansprüchen 19-29, **dadurch gekennzeichnet, dass** das erste und/oder das zweite Gerät Mittel zum Vorschlagen und/oder Darbieten einer Anzahl von Vorgängen umfasst, die für die Behandlung und Unterstützung des Patienten, zumindest teilweise eine spezifizierte/vorbestimmte Verordnung zu befolgen, relevant sind.

31. System nach den Ansprüchen 19-30, **dadurch gekennzeichnet, dass** die Dateninformationen eines oder mehrere von Folgendem betreffen:
- Medikamentenmenge,
- Medikamententyp,
- Körperfluidkonzentration,
- Zeitstempel,
- Nahrungsmenge,
- Messung von physikalischer Aktivität,
- Terminmitteilung,
- Bestandslogistik und
- Körpereigenschaften.

32. System nach den Ansprüchen 19-31, **dadurch gekennzeichnet, dass** die Dateninformationen zu mindestens einem gesendet werden, das ausgewählt ist aus der Gruppe:
- eines an ein paketvermitteltes Netzwerk angeschlossenen Zentralservers (76),
- eines Mobiltelefons,
- eines an ein paketvermitteltes Netzwerk angeschlossenen Clients und
- einer anderen Vorrichtung, die in der Lage ist, die empfangenen Dateninformationen zu empfangen, anzuzeigen und/oder zu verarbeiten.

33. System nach Anspruch 32, **dadurch gekennzeichnet, dass** die Dateninformationen am Zentralserver Angehörigen oder jeder beliebigen anderen relevanten dritten Partei zugänglich ist, indem eine Verbindung zwischen einem Computer (77; 78; 79) und dem Server aufgebaut wird.

34. System nach den Ansprüchen 32-33, **dadurch gekennzeichnet, dass** die Dateninformationen am Zentralserver automatisch an Angehörige und/oder jede beliebige relevante dritte Partei per E-Mail oder durch andere Nachrichtenformate an Computer und/oder Mobilkommunikationsendgeräte gesandt werden.

35. System nach den Ansprüchen 32-34, **dadurch gekennzeichnet, dass** die Dateninformationen am Zentralserver zusammen mit einer klinischen Studie eines vorbestimmten Produkts verwendet werden.

36. System nach den Ansprüchen 32-35, **dadurch gekennzeichnet, dass** die Dateninformationen am Zentralserver von einem Arzt verwendet werden, um zu ermitteln, wann eine Konsultation erforderlich ist.

## Revendications

1. Un procédé pour contrôler des informations de données entre deux appareils médicaux portables (101 ; 102), l'utilisation des appareils comprenant une première opération et une seconde opérations, lesdits appareils portables comprenant un premier appareil (101) pour exécuter la première opération et un second appareil (102) pour exécuter la seconde opération, chacun des appareils possédant des moyens pour un ou plusieurs d'entre : stockage, émission, réception, traitement et affichage d'informations de données, et les deux appareils possédant plusieurs positions en relation mutuelle en utilisation normale, **caractérisé en ce que** le procédé comprend les étapes suivantes :
• stockage automatique d'au moins des premières informations de données relatives à ladite première opération dans ledit premier appareil (101),
• stockage automatique d'au moins des secondes informations de données relatives à ladite seconde opération dans ledit second appareil (102), et
• émission automatique, via des moyens de communication à courte portée (117 ; 118), des informations de données relatives au moins auxdites première et seconde opérations entre lesdits premier et second appareils lorsque lesdits appareils sont positionnés mutuellement dans l'une desdites plusieurs positions en relation mutuelle,
ledit second appareil étant un dispositif d'administration de médicament.

2. Un procédé selon la revendication 1, **caractérisé en ce que** l'étape d'émission automatique d'informations de données entre lesdits premier et second appareils comprend un ou plusieurs d'entre :
• vérification pour chaque appareil d'un numéro d'identification individuel et unique d'appareil, et
• jumelage d'un premier appareil à un second appareil par liaison de leurs numéros d'identification individuels et uniques.

3. Un procédé selon l'une des revendications 1 à 2, **caractérisé en ce que** ledit premier appareil est une unité de capuchon protecteur.

4. Un procédé selon les revendications 1 à 3, **caractérisé en ce que** l'une des positions desdites plusieurs positions en relation mutuelle est obtenue lorsque ledit premier appareil est monté sur ledit second appareil.

5. Un procédé selon les revendications 1 à 4, **caractérisé en ce que** ledit premier appareil comprend un analyseur de fluide corporel intégré.

6. Un procédé selon les revendications 1 à 5, **caractérisé en ce que** ledit premier appareil comprend un récepteur sans fil intégré pour recevoir des informations de données d'un troisième appareil.

7. Un procédé selon la revendication 6, **caractérisé en ce que** ledit troisième appareil est choisi dans le groupe formé par :
• un mesureur de glucose sanguin en continu (CGM) disposé sur le corps du patient,
• un moniteur de pouls,
• une balance,
• et tout autre appareil apte à mesurer au moins un paramètre physiologique.

8. Un procédé selon les revendications 1 à 7, **caractérisé en ce que** ledit premier appareil est choisi dans le groupe formé par :
• un moniteur de lipides,
• un moniteur de pouls,
• un dispositif bistouri,
• un conteneur de stockage, et
• un moniteur de glucose sanguin (BGM).

9. Un procédé selon les revendications 1 à 8, **caractérisé en ce que** ledit second appareil est un dispositif d'administration d'insuline.

10. Un procédé selon les revendications 1 à 9, **caractérisé en ce que** lesdites informations de données sont émises en utilisant l'un des moyens choisis dans le groupe formé par :
• des moyens de communication optiques,
• des moyens de communication inductifs, et
• des moyens de communication électriques.

11. Un procédé selon les revendications 1 à 10, **caractérisé en ce que** lesdites première et seconde opérations sont choisies dans le groupe formé par :
• l'injection d'un médicament,
• la mesure d'un fluide corporel,
• l'administration d'un certain nombre de glucides, et
• l'exécution d'une activité physique.

12. Un procédé selon les revendications 1 à 11, **caractérisé en ce que** lesdits premier et/ou second appareils comprennent des moyens pour suggérer et/ou présenter un certain nombre d'opérations relatives audit traitement et aidant le patient à être au moins partiellement en conformité avec un régime spécifié/prédéterminé.

13. Un procédé selon les revendications 1 à 12, **caractérisé en ce que** lesdites informations de données sont en rapport avec une ou plusieurs d'entre :
• quantité de médicament,
• type de médicament,
• concentration de fluide corporel,
• horodatage,
• quantité de nourriture,
• mesure d'activité physique,
• notification d'un rendez-vous,
• logistique d'inventaire, et
• caractéristiques corporelles.

14. Un procédé selon les revendications 1 à 13, **caractérisé en ce que** lesdites informations de données sont émises vers au moins l'un choisi dans le groupe formé par :
• un serveur central (76) relié à un réseau de commutation par paquets,
• un téléphone mobile,
• un client connecté à un réseau de communication par paquets, et
• un autre dispositif capable de recevoir, montrer et/ou traiter des informations de données reçues.

15. Un procédé selon la revendication 14, **caractérisé en ce que** lesdites informations de données audit serveur central sont accessibles par des parents ou tous autres tiers appropriés par établissement d'une connexion entre un ordinateur (77 ; 78 ; 79) et ledit serveur.

16. Un procédé selon les revendications 14 à 15, **caractérisé en ce que** lesdites informations de données audit serveur central sont automatiquement émises vers les parents et/ou toutes autres parties tierces intéressés par courriel ou par d'autres formats de message vers des ordinateurs et/ou des terminaux de communication mobile.

17. Un procédé selon les revendications 14 à 16, **caractérisé en ce que** lesdites informations de données audit serveur central sont utilisées en liaison avec un essai clinique d'un produit prédéterminé.

18. Un procédé selon les revendications 14 à 17, **caractérisé en ce que** lesdites informations audit serveur central sont utilisées par un médecin afin de déterminer le moment où une consultation est nécessaire.

19. Un système pour contrôler des informations de données entre deux appareils portables (101 ; 102), l'utilisation des appareils comprenant une première opération et une seconde opérations, lesdits appareils portables comprenant un premier appareil (101) pour exécuter la première opération et un second appareil (102) pour exécuter la seconde opération, chacun des appareils possédant des moyens pour un ou plusieurs d'entre : stockage, émission, réception, traitement et affichage d'informations de données, et les deux appareils possédant plusieurs positions en relation mutuelle en utilisation normale, **caractérisé en ce que** :
• ledit premier appareil comprend des moyens de stockage pour stocker automatiquement au moins des premières informations de données relatives à ladite première opération,
• ledit second dispositif comprend des moyens de stockage pour stocker automatiquement au moins des secondes informations de données relatives à ladite seconde opération, et
• **en ce que** lesdits premier et second appareils comprennent des moyens de communication à courte portée (117 ; 118) pour émettre automatiquement des informations de données relatives à au moins l'une desdites première et seconde opérations entre lesdits premier et second appareils lorsque lesdits appareils sont mutuellement positionnés dans l'une d'entre lesdites plusieurs positions en relation mutuelle,
ledit second appareil étant un dispositif d'administration de médicament.

20. Système selon la revendication 19, **caractérisé en ce que** les moyens de stockage de chaque appareil comprennent un numéro individuel et unique d'identification d'appareil et **en ce que** les moyens de stockage d'au moins l'un des appareils comprennent des informations relatives au jumelage d'un premier appareil et d'un second appareil par liaison de leur numéro d'identification individuel et unique.

21. Système selon les revendications 19 à 20, **caractérisé en ce que** ledit premier appareil est une unité de capuchon protecteur.

22. Système selon les revendications 19 à 21, **caractérisé en ce que** l'une des positions desdites plusieurs positions en relation mutuelle est obtenue lorsque ledit premier appareil est monté sur ledit second appareil.

23. Système selon les revendications 19 à 22, **caractérisé en ce que** ledit premier appareil comprend un analyseur de fluide corporel intégré.

24. Système selon les revendications 19 à 22, **caractérisé en ce que** ledit premier appareil comprend un récepteur sans fil intégré pour recevoir des informations de données d'un troisième appareil.

25. Système selon la revendication 24, **caractérisé en ce que** ledit troisième appareil est choisi dans le groupe formé par :
• un mesureur de glucose sanguin en continu (CGM) disposé sur le corps du patient,
• un moniteur de pouls,
• une balance,
• et tout autre appareil apte à mesurer au moins un paramètre physiologique.

26. Système selon les revendications 19 à 25, **caractérisé en ce que** ledit premier appareil est choisi dans le groupe formé par :
• un moniteur de lipides,
• un moniteur de pouls,
• un dispositif bistouri,
• un conteneur de stockage, et
• un moniteur de glucose sanguin (BGM).

27. Système selon les revendications 19 à 26, **caractérisé en ce que** ledit second appareil est un dispositif d'administration d'insuline.

28. Système selon les revendications 19 à 27, **caractérisé en ce que** lesdites informations de données sont émises en utilisant l'un des moyens choisis dans le groupe formé par :
• des moyens de communication optiques,
• des moyens de communication inductifs, et
• des moyens de communication électriques.

29. Système selon les revendications 19 à 28, **caractérisé en ce que** lesdites première et seconde opérations sont choisies dans le groupe formé par :
• l'injection d'un médicament,
• la mesure d'un fluide corporel,
• l'administration d'un certain nombre de glucides, et
• l'exécution d'une activité physique.

30. Système selon les revendications 19 à 29, **caractérisé en ce que** lesdits premier et/ou second appareils comprennent des moyens pour suggérer et/ou présenter un certain nombre d'opérations relatives audit traitement et aidant le patient à être au moins partiellement en conformité avec un régime spécifié/prédéterminé.

31. Système selon les revendications 19 à 30, **caractérisé en ce que** lesdites informations de données sont en rapport avec une ou plusieurs d'entre :
quantité de médicament,
• type de médicament,
• concentration de fluide corporel,
• horodatage,
• quantité de nourriture,
• mesure d'activité physique,
• notification d'un rendez-vous,
• logistique d'inventaire, et
• caractéristiques corporelles.

32. Système selon les revendications 19 à 31, **caractérisé en ce que** lesdites informations de données sont émises vers au moins l'un choisi dans le groupe formé par :
• un serveur central (76) relié à un réseau de commutation par paquets,
• un téléphone mobile,
• un client connecté à un réseau de communication par paquets, et
• un autre dispositif capable de recevoir, montrer et/ou traiter des informations de données reçues.

33. Système selon la revendication 32, **caractérisé en ce que** lesdites informations de données audit serveur central sont accessibles par des parents ou tous autres tiers appropriés par établissement d'une connexion entre un ordinateur (77 ; 78 ; 79) et ledit serveur.

34. Système selon les revendications 32 à 33, **caractérisé en ce que** lesdites informations de données audit serveur central sont automatiquement émises vers les parents et/ou toutes autres parties tierces intéressés par courriel ou par d'autres formats de message vers des ordinateurs et/ou des terminaux de communication mobile.

35. Système selon les revendications 32 à 34, **caractérisé en ce que** lesdites informations de données audit serveur central sont utilisées en liaison avec un essai clinique d'un produit prédéterminé.

36. Système selon les revendications 32 à 35, **caractérisé en ce que** lesdites informations audit serveur central sont utilisées par un médecin afin de déterminer le moment où une consultation est nécessaire.
